# EUROPEAN PATENT APPLICATION

(11) **EP 4 506 396 A1**
(43) Date of publication of application: **12.02.2025**
(21) Application number: 23784762.9
(22) Date of filing: 04.04.2023
(51) Int. Cl.: C08J 3/14, C09K 11/06, A61Q 17/04, B01J 13/12, A61K 8/73, C09K 3/00, C09K 23/50

(54) **HOLLOW SPHERICAL PARTICLE**

(30) Priority: 04.04.2022 JP 2022062483
(71) Applicant: Kyoto University, Kyoto-shi, Kyoto 606-8501 (JP)
(72) Inventor: NISHIMURA, Hiroshi, Kyoto-shi, Kyoto 606-8501 (JP); TAKATA, Koji, Nanto-shi, Toyama 939-1503 (JP)
(74) Representative: Vossius & Partner Patentanwälte Rechtsanwälte mbB
(86) International application number: PCT/JP2023/014006
(87) International publication number: WO 2023/195476

(57) **Abstract**

Provided are a hollow spherical particle that has higher safety and can be prepared more easily. A hollow spherical particle that is a self-assembled body of lignin, the lignin having a content of β-O-4 ether type structure of 50% or more and a solubility in 60 to 80% ethanol at 25°C of 2 w/v% or more.

## Description

### Technical Field

The present invention relates to a hollow spherical particle and the like.

### Background Art

Hollow spherical particles, such as microcapsules and nanocapsules, are used in a wide range of industries, including agricultural chemicals, detergents, fabric softeners, fragrances, cosmetics, pharmaceuticals, latent heat storage materials (PCM), textiles, paints, and functional chemicals. Encapsulating a wide range of molecules, including drugs, into capsules is an important means of controlling the timing of the development of the molecules, performance, efficacy, properties, dispersibility, compatibility, weather resistance, and responsiveness to external stimuli. Most substrates of fine particles are petroleum-derived molecules, which are of concern as environmentally hazardous substances and endocrine-disrupting chemicals. Fine particles are difficult to recover after being released into the environment, and are part of the source of microplastics in soil, rivers, and oceans.

There have been reports of nanoparticles that use modified industrial lignin instead of petroleum-derived polymers (NPL 1); however, there are safety concerns because organic solvents that are harmful to living organisms are used in the production process thereof.

### Citation List

### Non-patent Literature

NPL 1: M. Lievonen et al. A simple process for lignin nanoparticle preparation, Green Chem. 2016, 18, 1416-1422 (https://pubs.rsc.org/en/content/articlelanding/2016/gc/c5gc01436 k)

### Summary of Invention

### Technical Problem

An object of the present invention is to provide a hollow spherical particle that has higher safety and can be prepared more easily.

### Solution to Problem

The present inventors conducted extensive research to achieve the above object. As a result, the inventors found that a hollow spherical particle that is a self-assembled body of lignin, the lignin having a content of β-O-4 ether type structure of 50% or more and a solubility in 60 to 80% ethanol at 25°C of 2 w/v% or more, could achieve the above object. After further research based on this finding, the present invention has been accomplished. The present invention encompasses the following embodiments.

Item 1. A hollow spherical particle that is a self-assembled body of lignin, the lignin having a content of β-O-4 ether type structure of 50% or more and a solubility in 60 to 80% ethanol at 25°C of 2 w/v% or more.

Item 2. The hollow spherical particle according to Item 1, wherein the lignin has a number average molecular weight (Mn) of 0.7 to 9 kDa, or the lignin has a weight average molecular weight (Mw) of 1 to 10 kDa.

Item 3. The hollow spherical particle according to Item 1 or 2, which has a number average particle size of 100 µm or less.

Item 4. The hollow spherical particle according to any one of Items 1 to 3, which has a number average particle size of 50 nm or more.

Item 5. The hollow spherical particle according to any one of Items 1 to 4, which has a void ratio of 60% or more.

Item 6. The hollow spherical particle according to any one of Items 1 to 5, which has a film thickness of 1 nm to 4000 nm.

Item 7. The hollow spherical particle according to any one of Items 1 to 6, comprising an active ingredient.

Item 8. The hollow spherical particle according to Item 7, wherein the active ingredient is at least one member selected from the group consisting of dyes, agricultural chemicals, fertilizers, pharmaceuticals, cosmetic ingredients, fragrances, fluorescent agents, paints, proteins, nucleic acids, lipids, hormones, sugars, glycosides, surfactants, adhesives, vitamins, coenzymes, minerals, cells, organelles, low-molecular-weight compounds, high-molecular-weight compounds, metal complexes, liposomes, and hydrogels.

Item 9. The hollow spherical particle according to any one of Items 1 to 8, wherein a liquid, a gas, or a solid (metal as a type) is enclosed therein.

Item 10. The hollow spherical particle according to Item 9, for use as a heat-insulating material, a compatibilizer, a catalyst, or a sensitizer.

Item 11. The hollow spherical particle according to any one of Items 1 to 10, wherein the lignin has a sulfur atom content of 0.15% or less.

Item 12. The hollow spherical particle according to any one of Items 1 to 11, wherein the lignin has a content of non-natural condensed structure of 1% or less.

Item 13. The hollow spherical particle according to any one of Items 1 to 12, wherein the lignin has a ratio of phenolic hydroxyl groups to alcoholic hydroxyl groups of 0.8 or less.

Item 14. The hollow spherical particle according to any one of Items 1 to 13, which exhibits long-wavelength fluorescence.

Item 15. The hollow spherical particle according to Item 14, wherein the long-wavelength fluorescence is fluorescence at 450 nm or more.

Item 16. An ultraviolet absorber comprising the hollow spherical particle according to any one of Items 1 to 15.

Item 17. A light resistance imparting agent comprising the hollow spherical particle according to any one of Items 1 to 15.

Item 18. An anti-Stokes fluorescent agent comprising the hollow spherical particle according to any one of Items 1 to 15.

### Advantageous Effects of Invention

The present invention can provide a hollow spherical particle that is superior in biocompatibility, biodegradability, non-toxicity, and amphiphilicity, and that can be prepared more easily.

### Brief Description of Drawings

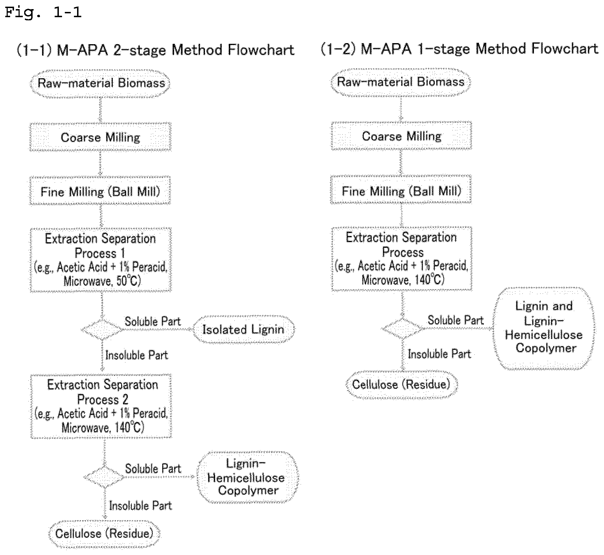

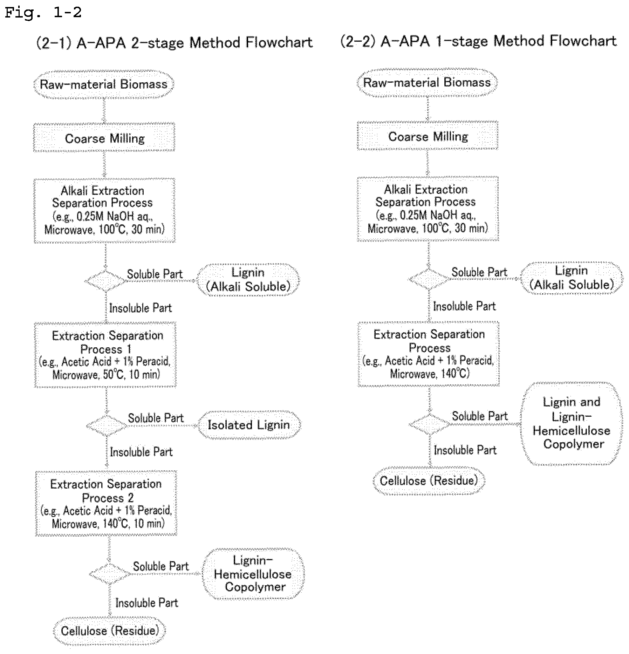

Fig. 1-1 shows flow charts of an M-APA method.
Fig. 1-2 shows flow charts of an A-APA method.
Fig. 2 shows microwave irradiation conditions for isolating lignocellulose components, power, temperature, and pressure profiles; and device specifications: Initiator+ 60 microwave synthesizer manufactured by Biotage, stirring speed: 600 rpm, Cooling: On, Absorption: High.
Fig. 3 is a two-dimensional NMR spectrum of a low-modified, high-purity lignin with a high ether-type content. This is a result of analysis of lignin isolated according to the M-APA method (microwave treatment at 50°C for 10 minutes with acetic acid-peracetic acid 1%) using *eucalyptus* as a starting material.
Fig. 4 shows the partial chemical structures of lignin interunit bonds and covalent bonds between lignin and hemicelluloses.
Fig. 5 shows two-dimensional NMR spectra a to d of lignin-hemicellulose copolymers, and enlarged views a' to d' of interunit bonds and saccharide regions. Lignin-hemicellulose copolymers obtained from hardwood (a, b) and softwood (c, d) according to the M-APA method were analyzed. The circles in the figure indicate LC bonds (ether type and ester type).
Fig. 6 is a synchro-fluorescence spectrum of APA lignin derived from bagasse (sugarcane) in the near-infrared wavelength region. This shows a result of synchro-scanning measurement for detecting a predetermined Stokes shift, that is, a result of the detection of fluorescence wavelength Em that is 80, 90, 150, 160, or 170 nm longer than excitation wavelength Ex (scanning of the excitation wavelength).
Fig. 7 shows a three-dimensional fluorescence spectrum of *Pinus densiflora* APA lignin in the near-infrared wavelength region. APA lignin obtained by treating wood flour of *Pinus densiflora* according to the M-APA method (microwaves at 50°C for 10 min with acetic acid + 0.2M hydrogen peroxide) was measured in an 80% acetonitrile aqueous solution (concentration: 0.1 wt%).
Fig. 8 is a graph showing a comparison between comparative synchro-scanning fluorescence spectra of commercial lignin (TCI alkaline lignin, Tokyo Chemical Industry Co., Ltd., blue line) and lignin (APA lignin, red line). A fluorescence longer than the excitation wavelength (plus 90 nm) was detected (the excitation wavelength scanned). Lignin (APA lignin) exhibited characteristic fluorescence in the long-wavelength region.

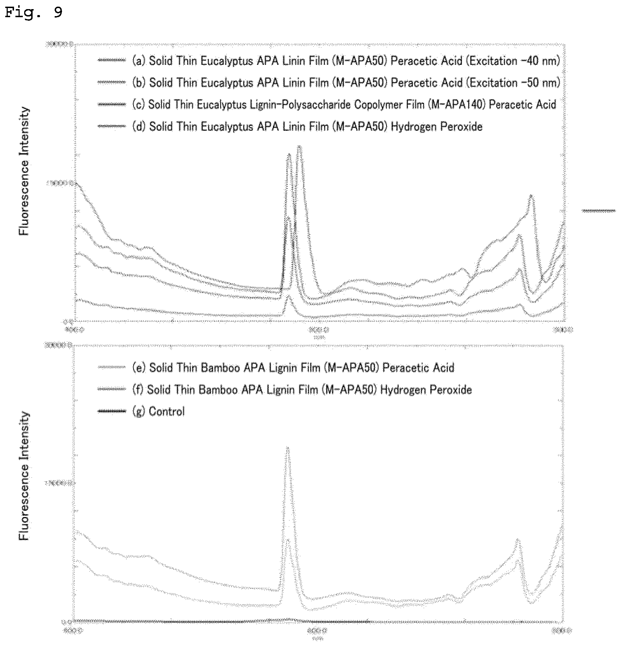

Fig. 9 shows synchronous anti-fluorescence spectra of solid thin lignin films and a solid thin lignin-polysaccharide copolymer film. Fig. 9 shows anti-fluorescence spectra (a to f) obtained by scanning an excitation wavelength and selectively detecting anti-Stokes-shifted fluorescence shorter than the excitation wavelength and control (g). The anti-Stokes shift was an excitation wavelength minus 50 nm; minus 40 nm only in spectrum (b). From the comparison between spectra (b) and (d) and the comparison between spectra (e) and (f), the anti-fluorescence intensity was found to be higher when peracetic acid, rather than hydrogen peroxide, was used as an oxidizing agent. The comparison of spectra (b) and (c) indicates that the APA lignin exhibits a higher anti-fluorescence intensity than the lignin-polysaccharide copolymer.
Fig. 10 shows UV absorption spectra of lignin and a lignin-hemicellulose copolymer. The vertical axis indicates absorbance, and the horizontal axis indicates wavelength (nm). For an explanation of the legend, see Table 8.
Fig. 11 shows a bright-field image of the hollow spherical particles obtained in Example 1.
Fig. 12 shows a fluorescence image of the hollow spherical particles obtained in Example 2.

### Description of Embodiments

In the present specification, the terms "comprise," "contain," and "include" include the concepts of comprising, containing, including, consisting essentially of, and consisting of.

In one embodiment, the present invention relates to a hollow spherical particle that is a self-assembled body of lignin (which may be referred to as "the lignin of the present invention" in the present specification), the lignin having a content of β-O-4 ether type structure of 50% or more and a solubility in 60 to 80% ethanol at 25°C of 2 w/v% or more (which may be referred to as "the hollow spherical particle of the present invention" in the present specification). The details are described below.

### 1. Lignin Isolation Method

The lignin of the present invention can be obtained by a method for isolating lignin from plant biomass, the method comprising step (A) of bringing a solution containing an organic acid and a peracid into contact with plant biomass (which may be referred to as "the isolation method of the present invention" in the present specification). The details are described below.

The isolation method of the present invention comprises step (A), whereby high-quality (low-condensation) lignin with a high content of the β-O-4 ether type structure among all of the binding modes between the monolignols can be obtained.

The plant biomass may be any plant biomass that contains lignin. Examples of the plant biomass include a plant body itself and a mechanically processed product of a plant body.

Examples of the plant body include softwood materials, hardwood materials, and non-wood materials. Specific examples include softwood materials of, for example, *Cryptomeria japonica,* pine, *Picea jezoensis, Larix kaempferi, Pinus thunbergii, Abies sachalinensis, Pinus parviflora, Taxus cuspidata, Thuja standishii, Picea polita, Picea alcokiana, Podocarpus macrophyllus, Abies firma, Chamaecyparis pisifera, Pseudotsuga japonica, Thujopsis dolabrata* var. *dolabrata, Thujopsis dolabrata* var. *hondae, Tsuga sieboldii, Tsuga diversifolia, Chamaecyparis obtusa, Taxus cuspidata, Cephalotaxus harringtonia, Picea jezoensis* var. *hondoensis,* yellow cedar *(Callitropsis nootkatensis),* Lawson cypress *(Chamaecyparis lawsoniana),* Douglas fir *(Pseudotsuga menziesii),* Sitka spruce *(Picea sitchensis), Pinus radiata,* eastern spruce, eastern white pine, western larch, western fir, western hemlock, and tamarack; hardwood materials of, for example, *Populus Tremuloides,* American black cherry, *Liriodendron tulipifera,* walnut, kaba-zakura, *Zelkova serrata,* sycamore, silver cherry, *Fraxinus mandshurica, Tectona grandis,* Chinese elm, Chinese maple, *Quercus, Fagus crenata,* hard maple, hickory, *Carya illinoinensis, Fraxinus americana,* white oak, white birch, red oak, acacia, and eucalyptus; and non-wood materials of, for example, *Oryza sativa, Saccharum officinarum,* barley, wheat, maize, pineapple, oil palm, *Hibiscus cannabinus,* cotton, alfalfa, *Phleum pratense,* bamboo, Sasa, bamboo, and sugar beet.

Examples of the mechanically processed product of a plant body include logs, rectangular lumbers, boards, solid wood, wood materials, engineered wood, laminated veneer lumbers, plywood, wooden board, particle board, fiber board, wood chips, particulate wood materials (e.g., chips, particles, and wood flour), fibrous wood materials, compressed materials, and crushed materials.

The plant biomass is preferably wood flour from the viewpoint of being able to improve the yield. The volume average particle size of wood flour is, for example, 500 µm or less, preferably 200 µm or less, more preferably 100 µm or less, even more preferably 50 µm or less, and still more preferably 20 µm or less. The lower limit of the average particle size may be any value and can be, for example, 1 µm, 2 µm, or 5 µm. By using wood flour with a relatively small particle size, a high yield can be achieved without the alkali treatment described below.

Wood flour can be obtained by pulverizing plant biomass. Pulverization can be performed following or in accordance with known methods, for example, by using various types of mills (e.g., ball mills and mixer mills).

The plant biomass is preferably an alkali-treated material of a plant body or an alkali-treated material of a mechanically processed product of a plant body from the viewpoint of being able to improve the yield. According to the present invention, the use of the alkali-treated material makes it possible to efficiently obtain the target product without performing pulverization treatment (even when the specific surface area is relatively low, as is the case with wood chips). The alkali-treated material can be obtained by subjecting a plant body or a mechanically processed product thereof to alkali treatment. Alkali treatment can improve the yield by causing loosening of the plant cell wall structure (reducing intermolecular interactions and cleaving hydrogen bonds) and by cleaving intramolecular ester bonds. This allows a high yield to be achieved without physical pulverization of plant biomass or with a low degree of physical pulverization of plant biomass, thereby reducing process costs. Furthermore, the yield can be improved by 1.2 to 2 times. Moreover, the cellulose fibers obtained through the alkali treatment have improved crystallinity.

Specifically, the alkali treatment is preferably, for example, step (X) of bringing the plant body or the mechanically processed product thereof into contact with an alkaline solution.

The alkaline solution may be any alkaline solution as long as the above object can be achieved. For example, the alkaline solution can be a solution with a pH of 12 to 15. The pH of the solution is preferably 13 to 14. The alkaline solution can also be, for example, a solution containing 0.3 to 2 mass% (preferably 0.7 to 1.5 mass%) of an alkali metal hydroxide (sodium hydroxide, potassium hydroxide, etc.).

The amount of the alkaline solution for use may be any amount as long as the above object can be achieved. The alkaline solution can be used in an amount of, for example, 3 to 20 mL, and preferably 5 to 12 mL, per 1 g of the plant body or the mechanically processed product thereof.

The contact mode between the plant body or the mechanically processed product thereof and the alkaline solution may be any mode. From the viewpoint of, for example, treatment efficiency, it is preferable to immerse the plant body or the mechanically processed product thereof in the alkaline solution.

The temperature of the alkaline solution may be any temperature as long as the above object can be achieved. The temperature of the alkaline solution can be, for example, 20 to 150°C, preferably 50 to 120°C, and more preferably 80 to 120°C. When heating is performed, examples of the heating method include an internal heating method using microwave heating, and an external heating method using an oil bath etc. Preferred is an internal heating method using microwave heating.

The treatment time in step (X) is not limited as long as the above object can be achieved. The treatment time is, for example, 5 to 120 minutes, and preferably 15 to 60 minutes.

After step (X), the soluble part and the insoluble part are separated, and the insoluble part can be subjected to step (A) as an alkali-treated material of a plant body or an alkali-treated material of a mechanically processed product of a plant body.

Plant biomasses can be used singly or in a combination of two or more.

The solution containing an organic acid and a peracid (an organic acid-peracid solution) may be any solution that contains an organic acid and a peracid, and that is capable of extracting and solubilizing at least one member selected from the group consisting of lignin, hemicelluloses, and lignin-polysaccharide complexes.

Examples of the organic acid include, but are not limited to, acetic acid, formic acid, glyoxylic acid, maleic acid, propionic acid, folic acid, isobutyric acid, valeric acid, isovaleric acid, pyruvic acid, oxalic acid, malonic acid, succinic acid, glutaric acid, ketoglutaric acid, adipic acid, lactic acid, tartaric acid, fumaric acid, oxaloacetic acid, malic acid, isocitric acid, citric acid, benzoic acid, phthalic acid, isophthalic acid, terephthalic acid, hemimellitic acid, trimellitic acid, trimesic acid, mellophanic acid, prehnitic acid, pyromellitic acid, mellitic acid, methanesulfonic acid, benzenesulfonic acid, p-toluenesulfonic acid, camphorsulfonic acid, p-toluenesulfinic acid, and benzenesulfinic acid. Among these, preferred are acetic acid, formic acid, glyoxylic acid, 3-oxopropanoic acid, 2-methyl-3-oxopropanoic acid, and the like, more preferred are acetic acid, formic acid, glyoxylic acid, and the like, and particularly preferred is acetic acid.

The organic acids can be used singly or in a combination of two or more.

The peracid may be any acid that contains a hydroperoxide group (-O-OH). Examples of peracids include organic peroxo acids such as percarboxylic acid, and persulfuric acid, percarbonic acid, perphosphoric acid, and peroxo-perhalogenic acid. Examples of percarboxylic acids include peracetic acid, performic acid, perbenzoic acid, and metachloroperbenzoic acid. Examples of peroxo-perhalogenic acid include peroxo-perchloric acid, peroxo-perbromic acid, and peroxo-periodic acid. In addition to the above, examples of peracids include hydrogen peroxide, lithium peroxide, sodium peroxide, potassium peroxide, sodium percarbonate, urea peroxide, sodium perborate, tert-butyl hydroperoxide, cumene hydroperoxide, di-tert-butyl peroxide, dimethyldioxirane, acetone peroxide, methyl ethyl ketone peroxide, and hexamethylene triperoxide diamine. Among these, preferred are organic peroxo acids, hydrogen peroxide, sodium percarbonate, urea peroxide, sodium perborate, and the like, more preferred are percarboxylic acids (in particular, peracetic acid), hydrogen peroxide, and the like, and particularly preferred is hydrogen peroxide.

The peracids can be used singly or in a combination of two or more.

The organic acid content in the organic acid-peracid solution is, for example, 5 to 30 mol/L, preferably 8 to 25 mol/L, and more preferably 10 to 20 mol/L. The organic acid content in the organic acid-peracid solution is preferably 3 to 10 g, more preferably 4 to 8 g, and even more preferably 5 to 7 g, per 1 g of the plant biomass to be brought into contact with the organic acid-peracid solution.

The peracid content in the organic acid-peracid solution is, for example, 0.05 to 3 mol/L, preferably 0.08 to 2.5 mol/L, and more preferably 0.1 to 2 mol/L. The peracid content in the organic acid-peracid solution is preferably 0.03 to 0.1 g, more preferably 0.04 to 0.08 g, and even more preferably 0.05 to 0.07 g, per 1 g of the plant biomass to be brought into contact with the organic acid-peracid solution.

The contact mode between the plant biomass and the organic acid-peracid solution may be any mode. From the viewpoint of, for example, treatment efficiency, it is preferable to immerse the plant biomass in the organic acid-peracid solution.

The temperature of the organic acid-peracid solution may be any temperature as long as lignin alone can be selectively extracted and solubilized. The temperature of the organic acid-peracid solution is, for example, 15 to 90°C (preferably 40 to 90°C). By setting the temperature to be approximately within the above range, high-quality (low-condensation) lignin with a high content of the β-O-4 ether type structure among all of the binding modes between the monolignols can be obtained. For example, when the starting material is wood-based biomass, lignin alone is more likely to be selectively obtained by performing step (A) using the organic acid-peracid solution at a temperature of, for example, 90°C or lower (preferably 40 to 90°C, more preferably 40 to 70°C, and even more preferably 40 to 60°C). For example, when the starting material is herbaceous biomass, lignin alone is more likely to be selectively obtained by performing step (A) using the organic acid-peracid solution at a temperature of, for example, 60°C or lower (preferably 15 to 60°C). When heating is performed, examples of the heating method include an internal heating method using microwave heating, and an external heating method using an oil bath etc. Preferred is an internal heating method using microwave heating. Rapid internal heating with microwaves enables quick and efficient pretreatment.

The treatment time in step (A) is not limited as long as the above object can be achieved. The treatment time is, for example, 1 to 60 minutes, preferably 1 to 30 minutes, and more preferably 5 to 20 minutes.

In step (A), microwave treatment is preferably performed while the biomass is immersed in the solution. This improves the lignin yield. The conditions for microwave treatment can be set as appropriate. For example, a commercially available microwave synthesizer can be used while stirring the solution. The microwave treatment time can be, for example, 1 to 30 minutes, and preferably about 10 minutes. The isolation method of the present invention can also easily be constructed as a continuous system as well as a batch system, since the microwave treatment, when performed, requires only a relatively short irradiation time.

In step (A), it is preferable to irradiate the plant biomass with microwaves or ultrasonic waves from the viewpoint of being able to promote penetration of the solution into the internal pits inside the plant cell wall and being able to attempt to improve the yield. The irradiation time is the same as described above.

After step (A), a separating solvent is added, if necessary, and then the soluble part and insoluble part are separated. The method of separating the soluble part from the insoluble part may be any method. Examples of usable methods include decantation, spin-down, and filtration. The separating solvent may be dioxane, ethanol, acetone, acetonitrile, DMSO, DMF, NMI, ethyl acetate, methanol, butanol, various other alcohols, acetic acid, water, and mixed solvents thereof. By using these, lignin is separated. Preferred are organic solvents of acetone, ethanol, and acetonitrile, and mixtures of 10 to 20 v/v% organic solvents in water, from the viewpoint of extraction efficiency, selectivity, and environmental burden.

The soluble part obtained in step (A) contains lignin. Thus, the soluble part can be obtained as lignin, either as is or through other treatments, if necessary.

Examples of the other treatments include the following. After step (A), water can be removed by evaporation and/or lyophilization, if necessary, to obtain the target product as a dry powder. If desalting is necessary, the concentrated solution may be extracted, for example, with alcohol or acetone, and the solvent may be distilled off. This allows the target product to be obtained as a dry powder.

As a novel process for producing paper pulp with low energy and no harmful effluents, the present invention can provide a novel method for pretreating plant biomass, the method being a low-temperature, mild process with sufficiently delignified polysaccharides, so that the recovered product contains a further reduced amount of inhibitory substances that cause problems in ethanol fermentation.

### 2. Lignin

The isolation method of the present invention makes it possible to obtain lignin characterized by at least having a content of the β-O-4 ether type structure of 50% or more and a solubility in 60 to 80% ethanol at 25°C of 2 w/v% or more.

In one embodiment, the present invention relates to lignin having a content of the β-O-4 ether type structure of 50% or more and a solubility in 60 to 80% ethanol at 25°C of 2 w/v% or more, or relates to lignin obtainable by the isolation method of the present invention.

The lignin of the present invention refers to lignin in a state isolated from biomass (including natural lignin). The binding modes in natural lignin are diverse. Representative examples of the binding modes in natural lignin include the following.

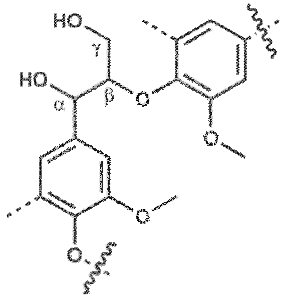

The above is called a β-O-4 bond.

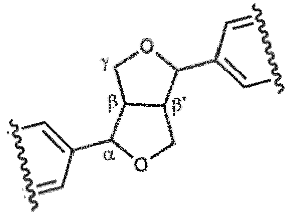

The above is called a resinol (β-β) bond.

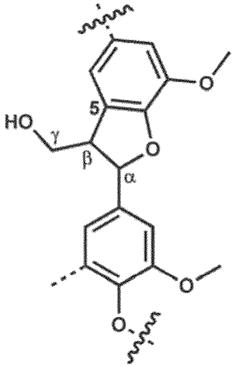

The above is called a phenylcoumaran (β-5) bond.

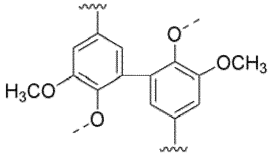

The above is called a 5-5 bond.

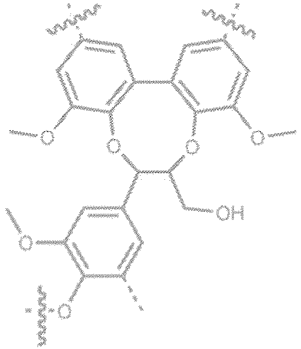

The above is called a dibenzodioxin (DBDO 5-5/4-O-β) bond.

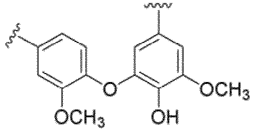

The above is called a 4-O-5 bond.

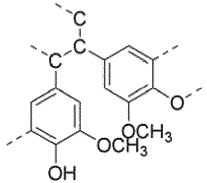

The above is called a β-1 bond.

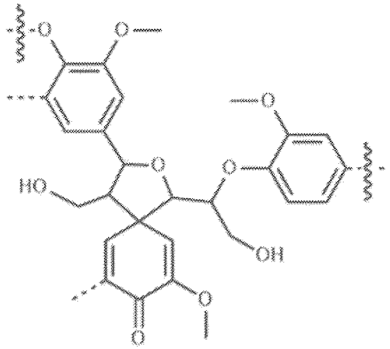

The above is called a spirodienone bond.

In natural lignin, the content of the β-O-4 ether type structure is highest among all of the binding modes between the monolignols, although it also varies depending on plant species. For example, in softwood, the content of the same is considered to be 40 to 60%. Further, in hardwood, for example, the content of the same in eucalyptus is considered to be about 45%.

In the lignin of the present invention, the content of the β-O-4 ether type structure is 50% or more, preferably 60% or more, more preferably 65 to 85%, and even more preferably 70 to 80%, among all of the binding modes between the monolignols.

In the lignin of the present invention, the content (%) of the β-O-4 ether type structure among all of the binding modes between the monolignols is preferably 1.2x% or more, more preferably 1.4x% or more, and even more preferably 1.6x% or more, with the content of the same in the lignin in the biomass used as a starting material being defined as x%. In the above, the upper limit is preferably 2x%.

The content of the β-O-4 ether type structure among all of the binding modes between the monolignols in lignin is specifically measured as follows. A sample is dissolved in deuterated dimethyl sulfoxide (DMSO-d₆) to 1 to 10 wt%, and the NMR spectrum is obtained using a Bruker Avance III 600 MHz NMR Spectrometer equipped with a low-temperature probe. The content of the β-O-4 ether type structure can be estimated from the signal volume in the HSQC (Heteronuclear single-quantum correlation spectroscopy) spectrum. The quantitative value in the two-dimensional NMR method is corrected according to a document (H. Okamura, H. Nishimura, T. Nagata, T. Kigawa, T. Watanabe, and M. Katahira, Accurate and molecular-size-tolerant NMR quantitation of diverse components in solution, Scientific Reports, 6, 21742, 2016) to calculate the accurate content.

The lignin of the present invention is characterized by having a solubility in 60 to 80% ethanol at 25°C of 2 w/v% or more. For this reason, by using the lignin of the present invention, a hollow spherical particle described below can be easily obtained without using solvents that are harmful to living organisms, and a hollow spherical particle with high stability can be obtained. The solubility is preferably 4 w/v% or more, more preferably 5 w/v% or more, even more preferably 6 w/v% or more, and still more preferably 7 w/v% or more.

The measurement method for the solubility is as follows. A sample is stirred and dissolved in ethanol at 25°C and the resulting product is separated into an ethanol-soluble part and ethanol-insoluble part by solid-liquid separation. An aqueous ethanol solution at 25°C (1/1 = ethanol/water) is added to the ethanol-insoluble part, and the mixture is stirred for dissolution to obtain an aqueous ethanol solution-soluble part. The aqueous ethanol solution-soluble part is mixed with the ethanol soluble part to obtain a sample solution. The amount of the aqueous ethanol solution added is adjusted so that the ethanol concentration of the sample solution is in the range of 60 to 80%. In the step of obtaining the soluble part, dispersion and solubilization are performed with an ultrasonic cleaner at 28 kHz for 3 min, if necessary. The maximum sample concentration at which the sample solution is visually transparent is defined as "solubility in 60 to 80% ethanol at 25°C."

In the lignin of the present invention, the constituent ratio of biphenyl-type lignin with a condensed structure is preferably 5% or less.

The lignin of the present invention has a number average molecular weight (Mn) of preferably 0.7 to 9 kDa, more preferably 1 to 5 kDa, and even more preferably 1.5 to 4 kDa.

The lignin of the present invention has a weight average molecular weight (Mw) of preferably 1 to 10 kDa, more preferably 1.5 to 8 kDa, and even more preferably 2 to 6 kDa.

In the present invention, the number average molecular weight (Mn) and weight average molecular weight (Mw) of lignin are specifically measured as follows. The molecular weight of lignin can be determined by size exclusion chromatography (SEC), i.e., gel permeation chromatography (GPC). A sample is dissolved in a solution of acetic anhydride and a base (e.g., pyridine) and reacted for 3 to 6 hours to obtain an acetylated product. The acetylated product is dissolved in tetrahydrofuran (THF) to a concentration of 2.0 mg/mL, and separation is performed by high-performance liquid chromatography. The columns for use are HZ-M columns (Tosoh Corporation, 150 mm × 4.6 mm id, 4 µm, three columns connected in series). The analysis is performed by injecting 10 µL of the sample at a column temperature of 40°C using tetrahydrofuran (THF) as the mobile phase at a flow rate of 0.35 mL/min. A mass calibration curve is prepared using standard polystyrene, Tosoh PStQuick C (molecular weights (MW): 2,110,000, 427,000, 37,900, and 5970), pinoresinol (MW: 352), and vanillin (MW: 152) to determine the Mw and Mn of the sample.

The lignin of the present invention has a polydispersity (Mw/Mn) of preferably 2.5 or less, and more preferably 2 or less.

Unlike industrial lignin using sulfur-containing components (sodium sulfide, sodium sulfate, sulfurous acid, etc.), the lignin of the present invention can be prepared without using these components. Therefore, in one embodiment, the lignin of the present invention does not contain any sulfur atoms or has a sulfur atom content of 0.15% or less (preferably 0.05% or less). The lignin of the present invention may contain trace amounts of sulfur atoms derived from sulfur-containing amino acids, sulfur components in the atmosphere, and the like.

The presence or absence and content of sulfur atoms can be determined and measured by elemental analysis using an X-ray fluorescence analyzer, SEM-EDX, XAFS, or the like.

The lignin of the present invention can be prepared under conditions that make it difficult to produce a non-natural condensed structure and a non-natural additional structure. Therefore, in one embodiment of the present invention, the lignin of the present invention has a content of the non-natural condensed structure of 1% or less.

The non-natural condensed structure is a structure in which the secondary and/or primary hydroxyl groups of a lignin molecule are condensed with other molecules, and is a structure that is newly formed in the lignin isolation step. The non-natural additional structure is a structure in which a lignin molecule is added with low-molecular-weight decomposition products produced by decomposition and/or the solvent and/or compounds (acid, base, alcohol, organic acid, carboxylic acid, aldehyde, amino acid, etc.) used in the isolation step, and is a structure that is newly formed in the lignin isolation step. The non-natural condensed structure and the non-natural additional structure are formed in the plant biomass pre-treatment, conversion, or lignin isolation step, and are easily formed under the following harsh conditions. The conditions include a strong acid or a strong base, and include multiple conditions of a high temperature (150°C or more) and/or a long time (1 hour or more) and/or a high pressure (1 atm or more). These structures are also formed by chemical and mechanochemical reactions under physical fine grinding conditions using a ball mill or bead mill.

The condensed structure restricts the degree of freedom of lignin oligomers in the solvent, which reduces molecular dispersibility, self-assembling properties, and fine particle formation ability. The lignin used in the present invention has an extremely low content of the condensed structure, and thus has excellent dispersibility, water affinity, self-assembling properties, hydrogen bond formation ability, fine particle formation ability, and hollow spherical capsule formation ability. In addition, the characteristic and advantage of having hydroxyl groups, which serve as the base for various derivatization and functionalization of fine particles, can be more effectively obtained. Examples of the non-natural condensed structure include the following structures, and the 5-5 bond structure and 4-O-5 bond structure mentioned above.

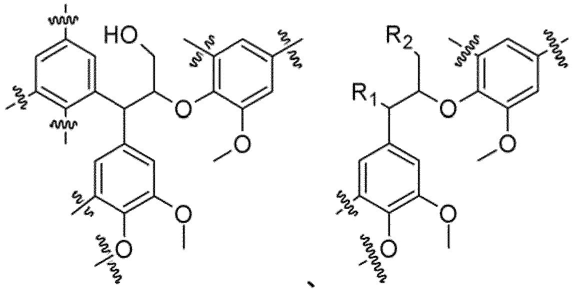

wherein R1 and/or R2 represents an alkyl group, -O-RX wherein RX represents an organic group (e.g., another structural part in lignin (benzene ring etc.)), or a hydroxyl group, excluding the case where R1 and R2 are both hydroxyl groups.

The contents of the non-natural condensed structure and the non-natural additional structure can be measured according to the method of Reference Test Example 1-6-2 described below, and can be calculated as the ratio per 100 aromatic rings of lignin.

The lignin of the present invention can be prepared under conditions that make it difficult to produce phenolic hydroxyl groups. Therefore, in one embodiment of the present invention, the lignin of the present invention has a ratio of phenolic hydroxyl groups to alcoholic hydroxyl groups of 0.8 or less (preferably 0.6 or less, more preferably 0.5 or less, even more preferably 0.4 or less, still more preferably 0.3 or less, and particularly preferably 0.2 or less). For the same reason, in one embodiment of the present invention, in the lignin of the present invention, the proportion of phenolic hydroxyl groups to all hydroxyl groups is 30% or less (desirably 20% or less). Fewer phenolic hydroxyl groups indicate an aromatic polymer with fewer branches, and by satisfying the above range, the particle formability and self-assembling properties are further improved.

The various hydroxyl groups can be quantified according to Reference Test Example 5 described below.

### 3. A hollow spherical particle

The hollow spherical particle of the present invention is not particularly limited as long as they are a self-assembled body of the lignin of the present invention and have a hollow spherical shape. Self-assembling occurs easily, and the conditions for inducing self-assembling can be appropriately set. For example, the lignin of the present invention can be dispersed in an aqueous solution and self-assembled to form a hollow spherical particle.

Specifically, in the above, self-assembling can be induced by gradually adding water to a sample dissolved in an organic solvent (e.g., alcohol) system to form a hollow spherical particle. The final water content is about 95%, but once capsules are formed, they become stable in water. Alternatively, capsules can be formed by dissolving a sample in alcohol-containing water etc., followed by natural evaporation. This results in the formation of capsules of sub-micron order with high uniformity.

Conventional lignin (commercially available industrial lignin) does not exhibit dispersibility or solubility in hydrophilic solvents, such as ethanol, and water-mixed solvents. The lignin of the present invention exhibits dispersibility and solubility in hydrophilic solvents and water-mixed solvents. Therefore, the use of the lignin of the present invention results in a hollow spherical particle that is safer for living organisms.

The hollow spherical particle of the present invention contains hollow portions, and the number of hollow portions is not particularly limited. The hollow spherical particle of the present invention can be a mononuclear type with one hollow portion, and can also be a polynuclear type with a plurality of hollow portions.

The self-assembled body of the lignin of the present invention exhibits fluorescence in the long-wavelength region. Specifically, the self-assembled body of the lignin of the present invention exhibits fluorescence in the wavelength range of 400 nm to 700 nm. Further, in the self-assembled body of the lignin of the present invention, in particular, fluorescence shifted to significantly shorter wavelength compared with the excitation wavelength, i.e., anti-Stokes fluorescence, is observed. Anti-Stokes fluorescence is observed even with an excitation light source weaker than a conventional powerful laser light source, such as a xenon lamp. For this reason, the hollow spherical particle of the present invention can be used as an anti-Stokes fluorescent agent. In this case, for example, the hollow spherical particle of the present invention can be used as an anti-Stokes fluorescent paint, ink, and identifier, as well as an upconversion/wavelength conversion molecular material.

The hollow spherical particle of the present invention is stably dispersed in an aqueous solution, and further can maintain the above fluorescence even in a dried solid state.

The hollow spherical particle of the present invention has a number average particle size of 100 µm or less, for example. In a preferred embodiment of the present invention, the hollow spherical particle of the present invention has a number average particle size of 0.7 µm or more, for example. The number average particle size is preferably 50 nm to 100 µm, more preferably 0.7 to 70 µm, even more preferably 1 to 50 µm, still even more preferably 1 to 40 µm, and particularly preferably 1 to 30 µm.

The hollow spherical particle of the present invention can have a high void ratio. The void ratio of the hollow spherical particle of the present invention is, for example, 60% or more, preferably 70% or more, more preferably 75% or more, and even more preferably 80% or more. The void ratio is measured according to the method described in Example 1.

In one embodiment of the present invention, the hollow spherical particle of the present invention preferably has a certain degree of film thickness. The film thickness of the hollow spherical particle of the present invention is preferably 1 to 4000 nm, more preferably 10 to 4000 nm, even more preferably 50 to 2000 nm, and still more preferably 100 to 1000 nm. The film thickness is measured according to the method described in Example 1.

The hollow spherical particle of the present invention can be used, for example, as a carrier for transporting substances by incorporating an active ingredient in their hollow portions. This also makes it possible to impart solubility resistance, compatibility, light resistance, and the like to the active ingredient. The active ingredient can be selected from a wide range. Examples include agricultural chemicals, fertilizers, pharmaceuticals, cosmetic ingredients, fragrances, fluorescent agents, paints (e.g., vanillin, limonene, and menthol), proteins, nucleic acids, lipids, hormones, sugars, glycosides, surfactants, adhesives, vitamins, coenzymes, minerals, cells, organelles, low-molecular-weight compounds (e.g., signaling substances such as pheromones; nitrogen-containing compounds such as alkaloids, monoterpenes, and sesquiterpenes, and isoprenoids such as terpenoids; and phenylpropanoids), high-molecular-weight compounds, metal complexes, liposomes, hydrogels, and the like.

Specifically, the hollow spherical particle of the present invention can be used as a drug carrier (for DDS). More specifically, the hollow spherical particle of the present invention can be used as organic capsules as drug carriers. Since they are natural products and are expected to have biocompatibility, they are useful as drug carriers.

In addition, specifically, the hollow spherical particle of the present invention can also be used as ultraviolet-absorbing capsules for the purpose of imparting light resistance to protect the encapsulated compound from ultraviolet rays. Since the absorption range includes UV-A, the hollow spherical particle of the present invention is useful as ultraviolet-absorbing materials for use in skin care products such as sunscreens. Such an ultraviolet absorption effect is presumed to be due to intermolecular interactions such as π-π stacking of aromatic lignin.

In the hollow spherical particle of the present invention, a liquid, a gas, or a solid (metal as a type) may be enclosed therein. In this case, the hollow spherical particle of the present invention can be used, for example, as a heat-insulating material, a compatibilizer, a catalyst, or a sensitizer.

The state of presence of the active ingredient, solid, etc. is not particularly limited as long as they are held in contact with the hollow spherical particle. For example, they may be present in the hollow portions of the hollow spherical particle, held by or attached to the constituent component (lignin) of the hollow spherical particle, or held by or attached to the outer surface of the hollow spherical particle.

The hollow spherical particle of the present invention exhibits long-wavelength fluorescence. This is due to the formation of intermolecular and intrapolymer associations of conjugated systems caused by the self-assembling of lignin, known as J-associates, and results in long-wavelength fluorescence due to exciplex formation, preferably fluorescence at 450 nm or more, more preferably fluorescence at 480 nm or more, and even more preferably fluorescence at 500 to 600 nm (specifically, e.g., Ex 515 nm/Em 535 nm), and large Stokes shift fluorescence of 100 nm or more (e.g., Ex 410 nm/Em 670 nm).

### Examples

The present invention will be described in detail below based on Examples. However, the present invention is not limited to these Examples.

### Reference Test Example 1: Isolation of Lignin, Lignin-Polysaccharide Complex, Hemicellulose, Cellulose-Hemicellulose Complex, and Cellulose

Lignin, lignin-polysaccharide complexes, hemicelluloses, cellulose-hemicellulose complexes, and cellulose were isolated from biomass according to an M-APA method, an A-APA method, or these methods with altered conditions. The yield, the physical properties, etc. of the isolated products were measured. The isolation methods according to the present invention are capable of isolating soluble lignin, lignin-polysaccharide complexes, hemicelluloses, cellulose-hemicellulose complexes, and cellulose from natural polymers in biomass with high efficiency, and includes step (A) of bringing a solution containing an organic acid and a peracid into contact with plant biomass. Specifically, the isolation was performed as described below.

### Reference Test Example 1-1: Preparation of Finely Milled Wood Flour

A fine powder (finely milled wood flour) with a volume average particle size of 10 µm (1 to 100 µm) was prepared from wood flour of hardwood (*eucalyptus* or *Fagus crenata*), softwood (*Pinus densiflora, Cryptomeria japonica,* or *Chamaecyparis obtusa*), or a herbaceous plant (Bagasse (sugarcane) or bamboo) by using a ball mill or bead mill method.

An example of preparation methods using a ball mill is described below. A planetary ball mill (P-6, Fritsch GmbH) was used. 2 g of wood flour and 100 g of zirconia beads with a diameter of 3 mm were placed in an 80-cc agate container and sealed in two overpots. After the overpots were vacuumed for 20 minutes, nitrogen gas (inert gas) was injected. A cycle composed of milling for 1 minute and a pause for 1 minute and 20 seconds was performed 180 times at 550 rpm (3 hours in total). The operation under the above conditions provides a fine wood flour while limiting oxidation during milling and denaturation due to heating.

### Reference Test Example 1-2: Preparation of Wood Flour

1 g of wood flour or wood chips (10 mm × 10 mm × 1 mm) of hardwood (*eucalyptus* or *Fagus crenata*), softwood (*Pinus densiflora, Cryptomeria japonica,* or *Chamaecyparis obtusa*), or a herbaceous plant (Bagasse (sugarcane) or bamboo) was placed in the left and right two pots of a mixer mill (MM301, Retsch). Under the conditions of a grinding time of 5 minutes and a frequency of 1/15 sec, milling was performed, thereby obtaining a powder (wood flour) with a volume average particle size of 0.1 mm.

### Reference Test Example 1-3: M-APA Method (Milling-Acid-PerAcid Method)

For a starting material, the finely milled wood flour (average particle size: 10 µm) obtained in Reference Test Example 1-1 was used. 2 g of finely milled wood flour and 10 mL of a solution containing acetic acid and peracetic acid (composition: 17.4M acetic acid 99%, 0.14M peracetic acid less than 1%, 0.01M hydrogen peroxide, and a trace amount of water) were placed in a vial for the Initiator and irradiated with microwaves for 10 minutes at 50°C, initial power of 400 W, and stirring speed of 600 rpm with an Initiator+ 60 microwave synthesizer (Biotage). After the soluble part was treated with microwaves, 10 mL of a separating solvent (acetone) was added, followed by separation by spin-down (RCF 8000 × g, 5 min). The insoluble part was extracted with 10 mL of a separating solvent (acetone) and washed three times, and combined with the soluble part. This soluble lignin is referred to as acid peracid lignin (APA lignin).

The insoluble part was subjected to the same extraction operation with the microwave heating condition changed to 140°C as the second step. The soluble part is referred to as a lignin-hemicellulose copolymer. An insoluble part containing cellulose as a main component was obtained.

### Reference Test Example 1-4: A-APA Method (Alkali-Acid-PerAcid Method)

For a starting material, the wood flour (average particle size: 0.1 mm) obtained in Reference Test Example 1-2 was used. A 0.25M sodium hydroxide solution was prepared (1N diluted 4 times). 2.4 mL of the 0.25 M sodium hydroxide solution was added to 0.3 g of wood flour, followed by microwave extraction treatment (100°C, 30 min). A rough indication of the required amount of an alkali is 1 mmol of NaOH per gram of wood flour.

After microwave treatment, the soluble part and the insoluble part can be easily separated by decantation, spin-down, or filtration. Extraction with acetone (separating solvent) was additionally performed three times, and the extract was combined with the soluble part. From the insoluble part, acid peracid lignin (APA lignin) and a lignin-hemicellulose copolymer were obtained as soluble lignin according to the same extraction method as the M-APA method of Reference Test Example 1-3. An insoluble part containing cellulose as a main component was obtained.

### Reference Test Example 1-5: Study of Conditions

Instead of acetic acid, other organic acids (formic acid, glyoxylic acid) were used for the study. Additionally, another peracid (hydrogen peroxide) was used instead of peracetic acid. For another peracid (hydrogen peroxide), the composition was changed as follows: acetic acid 12M, and hydrogen peroxide concentrations shown in the following table.

The study was conducted by changing the temperature at which plant biomass or an insoluble part comes in contact with an organic acid and a peracid to a temperature ranging from room temperature to 180°C. Additionally, at this time, the heating method was also changed from the internal heating method using microwave heating to an external heating method using an oil bath in conducting the study.

### Reference Test Example 1-6: Analysis and Measurement Method

Lignin, lignin-polysaccharide complexes, and cellulose were analyzed and measured according to the following methods.

### Reference Test Example 1-6-1: Measurement Method for Molecular Weight (Mn, Mw, and Degree of Polydispersity)

The molecular weight was determined by size exclusion chromatography (SEC), i.e., gel permeation chromatography (GPC). A sample was dissolved in a solution of acetic anhydride and a base (e.g., pyridine) and reacted for 3 to 6 hours to obtain an acetylated product. The acetylated product was dissolved in tetrahydrofuran (THF) to a concentration of 2.0 mg/mL, and separation was performed by high-performance liquid chromatography (high-pressure gradient SEC analysis system manufactured by Shimadzu Corporation). The columns for use were HZ-M columns (Tosoh Corporation, 150 mm × 4.6 mm id, 4 µm, three columns connected in series). The analysis was performed by injecting 10 µL of the sample at a column temperature of 40°C using tetrahydrofuran (THF) as the mobile phase at a flow rate of 0.35 mL/min. A mass calibration curve was prepared using standard polystyrene, Tosoh PStQuick C (molecular weights (MW): 2,110,000, 427,000, 37,900, and 5970), pinoresinol (MW: 352), and vanillin (MW: 152) to determine the weight average molecular weight Mw and the number average molecular weight Mn of the sample. The degree of polydispersity was determined as Mw/Mn.

### Reference Test Example: 1-6-2: Method for Calculating Constituent Ratio of Binding Units in Molecule

Samples were dissolved in deuterated dimethyl sulfoxide (DMSO-d₆) to 1 to 10 wt%, and their NMR spectra were obtained using a Bruker Avance III 600 MHz NMR Spectrometer equipped with a low-temperature probe. The constituent ratio of the interunit binding units of lignin was calculated from the volume of signals on the spectra of heteronuclear single-quantum correlation spectroscopy (HSQC). The constituent ratio was calculated as a ratio per 100 aromatic rings of lignin. The content of the ether binding unit was estimated from the sum of the integrated values of the C-H correlation signal at the β position (δH/δC = the region of 4.0 ± 0.2 ppm/85.6 ± 1.2 ppm, δH/δC = the region of 4.3 ± 0.2 ppm/83.0 ± 0.7 ppm). The quantitative value in the two-dimensional NMR method was corrected according to the TAF method described in a document (H. Okamura, H. Nishimura, T. Nagata, T. Kigawa, T. Watanabe, and M. Katahira, Accurate and molecular-size-tolerant NMR quantitation of diverse components in solution, Scientific Reports, 6, 21742, 2016) to calculate the accurate content.

### Reference Test Example 1-6-3: Method for Calculating Constituent Ratio of Lignin and Polysaccharides

The sum of integrated values of the C-H correlation signals at the anomeric position (first position) of polysaccharides was calculated according to the NMR method described above and corrected according to the TAF method, followed by calculating the ratio per 100 aromatic rings of lignin.

### Reference Test Example 1-6-4: Method for Calculating Constituent Ratio of Lignin-polysaccharide Copolymerization Point

In the same manner as described above, the sum of integrated values of the C-H correlation signals derived from the binding units of the LCα ether and the LCα ester (LC ether, δH/δC = the region of 4.50 ± 0.15 ppm/80.1 ± 1 ppm; LC ester, δH/δC = the region of 5.88 ± 0.15 ppm/74.0 ± 1.5 ppm) was calculated according to the 2D HSQC NMR method and corrected according to the TAF method, followed by calculating the ratio per 100 aromatic rings of lignin. In the A-APA method, although some of the ester bonds are hydrolyzed by an alkali treatment, an LCα ester-type lignin-hemicellulose copolymer can also be obtained.

### Reference Test Example: 1-6-5: Method for Calculating Yield from Raw Material Biomass

Each of the obtained components was confirmed with a pH meter, pH test paper, or peracid test paper (potassium iodide starch paper), and then neutralized and subjected to peracid quenching as necessary. Neutralization was performed by adding 1N HCl or 1N NaOH in small amounts stepwise. Peracid quenching was performed by adding a 1M aqueous sodium sulfite solution dropwise. The organic solvent was distilled off by using an evaporator. Subsequently, a dry powder was obtained by lyophilization. When a salt was produced by neutralization, the concentrated solution obtained after distillation of the organic solvent was re-extracted with a separating solvent (acetone) and desalted. Thereafter, the solvent was distilled off again with an evaporator, and lyophilization was performed, thereby obtaining a dry powder. Each of the obtained components was weighed, and the yield per raw material biomass was calculated.

### Results

A polymer with a desirable molecular structure, molecular weight, and yield can be obtained under the following conditions. Tables 1 to 7 show the results.

Table 1 shows the peracid conditions and the yield of each component of polymeric lignocellulose in the two-stage microwave extraction reaction according to the M-APA method (wt%, raw-material biomass ratio, raw material: *eucalyptus*).

**Table 1**

| Peracid concentration conditions | Peracetic acid 1% 0.14 mol/L | H₂O₂ 0.4% 0.11 mol/L | H₂O₂ 3.2% 1.0 mol/L | H₂O₂ 4.2% 1.4 mol/L |
|---|---|---|---|---|
| APA lignin | 7 | 6 | 9 | 11 |
| Lignin-hemicellulose copolymer | 12 | 11 | 14 | 25 |
| Residual cellulose | 79 | 82 | 75 | 63 |
| Total yield | 98 | 98 | 98 | 99 |

Table 2 shows the yield of each component of polymeric lignocellulose of various kinds of biomass in the two-stage microwave extraction reaction according to the A-APA method (wt%, raw-material biomass ratio).

**Table 2**

| **Raw-material biomass plant species** | **Hardwood** | | **Softwood** | | | **Herbaceous plant** | |
|---|---|---|---|---|---|---|---|
| | *Eucalyptus* | *Fagus crenata* | *Pinus densiflora* | *Cryptomeria japonica* | *Chamaecyparis obtusa* | **Bagasse (sugarcane)** | **Bamboo** |
| **Weakly alkaline soluble lignin** | **21** | **22** | **26** | **20** | **19** | **33** | **32** |
| **APAlignin** | **5** | **5** | **4** | **5** | **6** | **4** | **5** |
| **Lignin-hemicellulose copolymer** | **32** | **23** | **14** | **14** | **12** | **12** | **15** |
| **Total soluble part** | **58** | **51** | **44** | **40** | **37** | **50** | **51** |
| **Residual cellulose** | **44** | **39** | **58** | **65** | **61** | **48** | **52** |
| **Total** | **102** | **90** | **103** | **105** | **98** | **98** | **104** |

Table 3 shows the peracid conditions and the molecular weight of each component of polymeric lignocellulose in the two-stage microwave extraction reaction according to the M-APA method (GPC analysis values of acetylated products).

**Table 3**

| *Eucalyptus* **peracetic acid 1%** | Mn | Mw | Mw/Mn |
|---|---|---|---|
| **APA lignin** | 3814 | 7927 | 2.1 |
| **Lignin-hemicellulose copolymer** | 4203 | 8513 | 2.0 |

| *Eucalyptus* H₂O₂ 0.4% | Mn | Mw | Mw/Mn |
|---|---|---|---|
| **APAlignin** | 3510 | 6490 | 1.8 |
| **Lignin-hemicellulose copolymer** | 4128 | 6758 | 1.6 |

| *Eucalyptus* H₂O₂ 3.2% | Mn | Mw | Mw/Mn |
|---|---|---|---|
| **APA lignin** | 3964 | 9064 | 2.3 |
| **Lignin-hemicellulose copolymer** | 3364 | 6224 | 1.9 |

| *Eucalyptus* H₂O₂ 4.2% | Mn | Mw | Mw /Mn |
|---|---|---|---|
| **APAlignin** | 3709 | 9124 | 2.5 |
| **Lignin-hemicellulose copolymer** | 1865 | 4291 | 2.3 |

Table 4 shows the yield of each component of polymeric lignocellulose of various kinds of biomass in the two-stage microwave extraction reaction according to the M-APA method (wt%, raw-material biomass ratio).

**Table 4**

| **Biomass species** | **Hardwood** | | **Softwood** | | **Herbaceous plant** | |
|---|---|---|---|---|---|---|
| | *Eucalyptus* | *Fagus crenata* | *Pinus densiflora* | *Cryptomeria japonica* | **Bagasse (sugarcane)** | **Bamboo** |
| **APAlignin** | **7** | **4** | **7** | **4** | **7** | **11** |
| **Lignin-hemicellulose copolymer** | **14** | **9** | **8** | **9** | **13** | **18** |
| **Residual cellulose** | **71** | **84** | **83** | **85** | **79** | **69** |
| **Total yield** | **92** | **97** | **98** | **97** | **99** | **98** |

Solvent: Acetic acid (containing 1% of peracetic acid in an amount of 0.14 mol/L)
Microwave Extraction Conditions: An APA lignin soluble part was obtained (50°C, 10 minutes, first stage); A lignin hemicellulose copolymer was obtained (140°C, 10 minutes, second stage).

Table 5 shows the yield of each component of polymeric lignocellulose of various kinds of biomass in the first-stage microwave extraction reaction according to the A-APA method (wt%, raw-material biomass ratio).

**Table 5**

| **Raw-material biomass plant species** | **Hardwood** | | **Softwood** | | | **Herbaceous plant** | |
|---|---|---|---|---|---|---|---|
| | *Eucalyptus* | *Fagus crenata* | *Pinus densiflora* | *Cryptomeria japonica* | *Chamaecyparis obtusa* | **Bagasse (sugarcane)** | **Bamboo** |
| **Weakly alkaline soluble lignin** | **22** | **22** | **25** | **19** | **18** | **35** | **32** |
| **APA lignin + lignin polysaccharide complex** | **30** | **26** | **13** | **21** | **19** | **12** | **19** |
| **Total soluble part** | **52** | **48** | **39** | **40** | **37** | **47** | **51** |
| **Residual cellulose** | **54** | **55** | **63** | **65** | **68** | **51** | **55** |
| **Total** | **106** | **103** | **101** | **105** | **105** | **98** | **106** |

Table 6 shows the molecular weight of each component of polymeric lignocellulose of various kinds of biomass obtained according to the A-APA method (GPC analysis values of acetylated products).

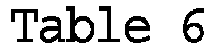

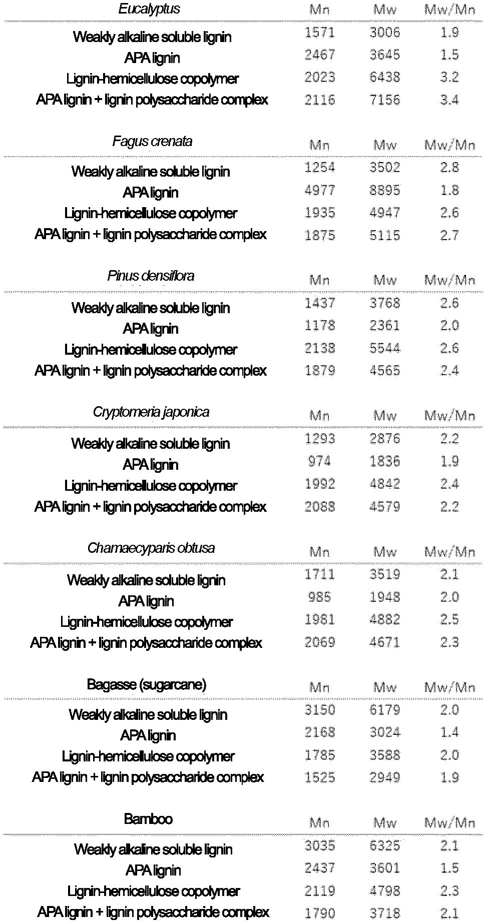

The study of conditions found that when the starting material is wood-based biomass, the temperature suitable for contact with an organic acid and a peracid is 40 to 90°C for obtaining soluble lignin; and that the temperature suitable for contact with an organic acid and a peracid is 100 to 160°C for obtaining a lignin-hemicellulose copolymer. The study of conditions also found that when the starting material is herbaceous biomass, the temperature suitable for contact with an organic acid and a peracid is from room temperature to 60°C for obtaining soluble lignin; and that the temperature suitable for contact with an organic acid and a peracid is 70 to 150°C for obtaining a lignin-hemicellulose copolymer.

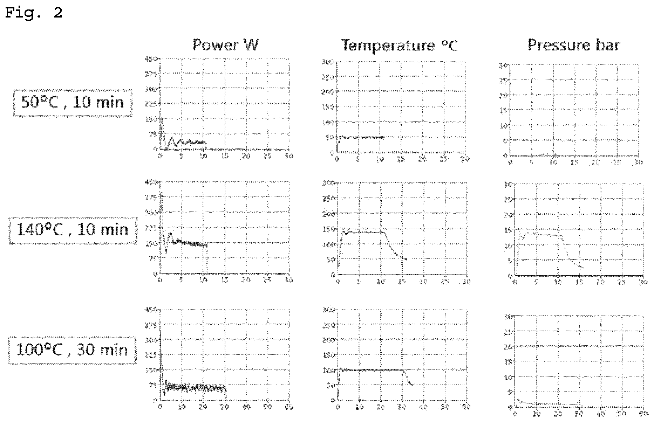

The study of conditions found that the heating time by microwave irradiation may be 5 to 30 minutes, and preferably 10 minutes. The net irradiation time was short. See Fig. 2 for the microwave profile, and data on temperature and pressure.

The study of conditions also found that the microwave heating resulted in a higher yield and a higher selectivity at low temperature in a short time with a low energy input than the external heating method. More specifically, microwave effects such as local heating, internal heating, electron transfer, and a chemical-permeation-promoting effect were observed.

The study of conditions also found that acetic acid, formic acid, and glyoxylic acid (organic acids) were all able to isolate lignin, and that acetic acid was most suitable from the viewpoint of yield, purity, and industrial application.

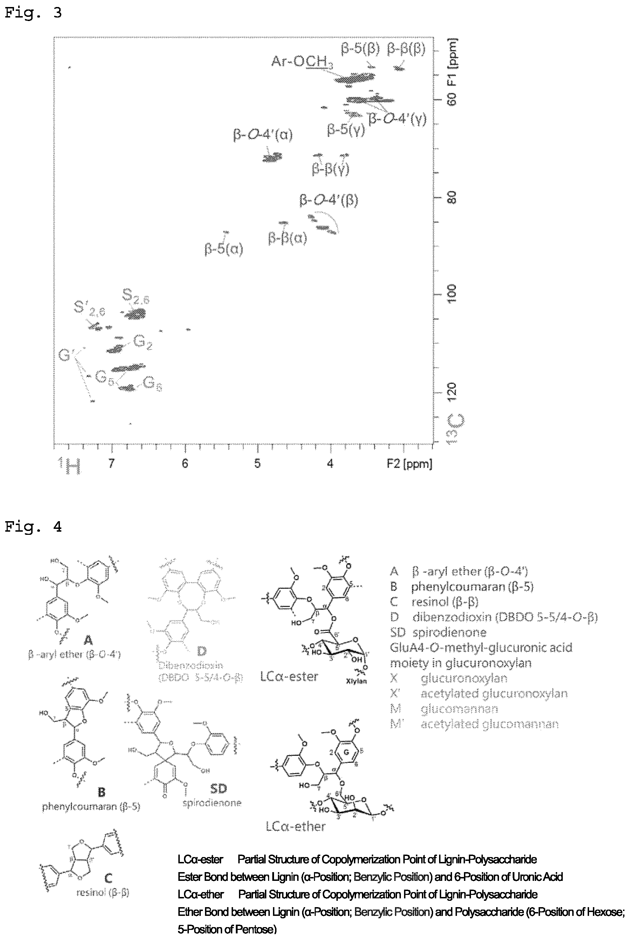

Fig. 3 shows the results of NMR in Reference Test Examples 1-6-2 and 1-6-3. Fig. 3 shows the results of analysis of lignin isolated from *eucalyptus* (starting material) according to the M-APA method (microwave treatment at 50°C for 10 minutes with acetic acid-peracetic acid 1%). Quantification of the proportion of interunit bonds of lignocellulose according to the TAF-NMR method found that the signal derived from lignin was 99%, and that the signal derived from polysaccharides (hemicelluloses) was 1%. The breakdown of the interunit bonds of lignin was the following: 81% of ether-type β-O-4, 5% of β-5, 11% of β-β, 1% of dibenzodioxocin, and 1% for the remainder. See Fig. 4 for the partial chemical structures of the interunit bonds of lignin and the covalent bonds of lignin-hemicellulose.

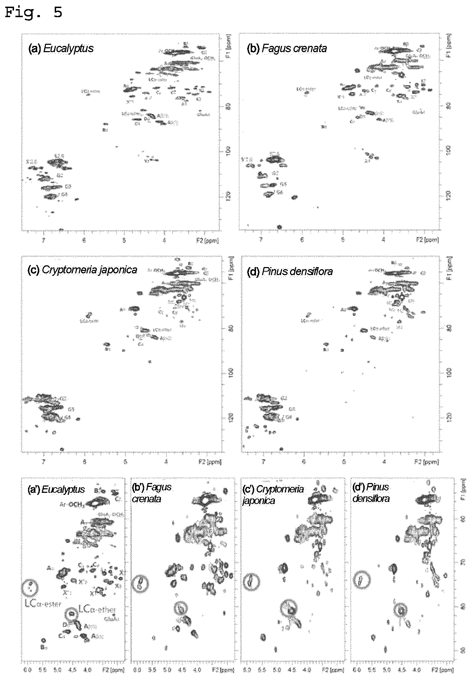

Fig. 5 shows the results of NMR in Reference Test Example 1-6-4. Fig. 5 shows the results of analysis of the lignin-hemicellulose copolymer isolated from individual plants (starting materials) according to the M-APA method (microwave treatment at 50°C for 10 minutes with acetic acid-peracetic acid 1%). The LC bonds (ether type and ester type) indicated by circles in the figure are a main branched structure present in a ratio equivalent to or higher than the abundance ratio of β-5 and β-β bonds, which are the main interunit bonds of lignin. The isolated lignin-hemicellulose copolymer is characterized in particular by the presence of an LCα-ester linkage.

### Reference Test Example 2: Solubility Measurement

The lignin and the lignin-hemicellulose copolymer obtained according to the method of Reference Test Example 1 or a method similar thereto were measured for the solubility in 60 to 80% ethanol at 25°C. Table 7 shows the details of the measurement samples. The abbreviations in Table 7 are as listed below.
PA: Peracetic Acid
HP: Hydrogen Peroxide
50: MW50 ACTN, treatment with microwaves at 50°C, high-quality polymeric lignin
140: MW50-Mw140 ACTN, after residues were treated with microwaves at 50°C, the residues were treated at 140°C (second stage), a lignin-hemicellulose copolymer
A50: An acetic acid-peracetic acid system, HP PA1% AA Mw50 ACN, high-quality polymeric lignin treated with microwaves at 50°C
H50: An acetic acid hydrogen peroxide system, HP 1M hydrogen peroxide, Mw50 ACN, high-quality polymeric lignin treated with microwaves at 50°C

**Table 7**

| **EA50** | *Eucalyptus* **Chip** | **Acetic Acid Peracetic Acid System PA1%AA Mw50 ACTN** |
|---|---|---|
| **EA140** | *Eucalyptus* **Chip** | **Acetic Acid Peracetic Acid System PA1%AA MW50-Mw140 ACTN** |
| **EH50** | *Eucalyptus* **Chip** | **Acetic Acid Hydrogen Peroxide System HP 1M Hydrogen Peroxide Mw50 ACN** |
| **BH50** | **Domestic** *Fagus crenata* **Sapwood** | **Acetic Acid Hydrogen Peroxide System HP 1M Hydrogen Peroxide Mw50 ACN** |
| **PH50** | **Domestic** *Pinus densiflora* **Sapwood** | **Acetic Acid Hydrogen Peroxide System HP 1M Hydrogen Peroxide Mw50 ACN** |
| **CH50** | **Domestic** *Cryptomeria japonica* **Sapwood** | **Acetic Acid Hydrogen Peroxide System HP 1M Hydrogen Peroxide Mw50 ACN** |
| **CH140** | **Domestic** *Cryptomeria japonica* **Sapwood** | **Acetic Acid Peracetic Acid System HP 1M Hydrogen Peroxide MW50-Mw140 ACTN** |
| **TA50** | **Domestic Bamboo:** *Phyllostachys edulis* **powder** | **Acetic Acid Peracetic Acid System PA1%AA Mw50 ACTN** |
| **TA140** | **Domestic Bamboo:** *Phyllostachys edulis* **powder** | **Acetic Acid Peracetic Acid System PA1%AAMW50-Mw140 ACTN** |
| **HP50** | **Domestic Bamboo:** *Phyllostachys edulis* **powder** | **Acetic Acid Hydrogen Peroxide System HP 1M Hydrogen Peroxide Mw50 ACN** |

The measurement method for the solubility is as follows. A sample was stirred and dissolved in ethanol at 25°C, and the resulting product was separated into an ethanol-soluble part and ethanol-insoluble part by solid-liquid separation. An aqueous ethanol solution at 25°C (1/1 = ethanol/water) was added to the ethanol-insoluble part, and the mixture was stirred for dissolution to obtain an aqueous ethanol solution-soluble part. The aqueous ethanol solution-soluble part was mixed with the ethanol soluble part to obtain a sample solution. The amount of the aqueous ethanol solution added was adjusted so that the ethanol concentration of the sample solution was in the range of 60 to 80%. In the step of obtaining the soluble part, dispersion and solubilization were performed with an ultrasonic cleaner at 28 kHz for 3 min, as necessary. The maximum sample concentration at which the sample solution was visually transparent was defined as "solubility in 60 to 80% ethanol at 25°C."

Table 8 shows the results. The results indicate that the lignin and lignin-hemicellulose copolymers obtained by the method of the present invention exhibit excellent ethanol solubility.

**Table 8**

| | Solubility |
|---|---|
| EA50 | 8 w/v% in 80% ethanol aqueous solution |
| EA140 | 8 w/v% in 80% ethanol aqueous solution |
| EH50 | 15 w/v% in 60% ethanol aqueous solution |
| BH50 | 20 w/v% in 60% ethanol aqueous solution |
| PH50 | 20 w/v% in 60% ethanol aqueous solution |
| CH50 | 8 w/v% in 70% ethanol aqueous solution |
| CH140 | 20 w/v% in 60% ethanol aqueous solution |
| TA50 | 8 w/v% in 80% ethanol aqueous solution |
| TA140 | 8 w/v% in 80% ethanol aqueous solution |
| HP50 | 15 w/v% in 60% ethanol aqueous solution |

### Reference Test Example 3: Preparation and Analysis of Self-assembled Body

A self-assembled body was prepared from lignin (sample) isolated from *eucalyptus* or bamboo (starting material) according to the M-APA method (microwave 50°C, 10 min-treatment, acetic acid-peracetic acid 1% or acetic acid-0.2M hydrogen peroxide) or a lignin-polysaccharide copolymer (sample) isolated from *eucalyptus* (starting material) according to the M-APA method (microwave 140°C, 10 min-treatment, acetic acid-peracetic acid 1% or acetic acid-0.2M hydrogen peroxide). Specifically, the preparation was performed as described below.

A DMSO solution with a sample concentration of 1 to 2 wt% was prepared and diluted to 0.01 to 0.02 wt% with a mixed solvent containing a separating solvent (acetonitrile or ethanol) and water (or a buffer) (1:1 or 2:1) to obtain a sample solution. 0.1 to 0.2 mL of the sample solution was applied to a glass slide or a cover glass, and the solvent was evaporated under atmospheric pressure to obtain a self-assembled body in the form of a thin film. Of the above conditions, when an equal amount of water (or a buffer) was added to a DMSO solution with a sample concentration of 1 wt%, and the solution was applied, a highly concentrated self-assembled body was obtained.

Subsequently, analysis was performed on the optical characteristics of the obtained self-assembled body (a solid thin lignin film or a solid thin lignin-polysaccharide copolymer film) and a comparative object (lignin obtained according to the APA method (APA lignin)). Specifically, the analysis was performed as follows.

The measurements were performed with the following.

UV-visible Absorption Spectrum: UV-2700 (manufactured by Shimadzu Corporation)
Fluorescence Spectrum: Spectrofluorometer RF-6000 (manufactured by Shimadzu Corporation)
Fluorescence Microscope Measurement: All-in-One Fluorescence Microscope BZ-X810 (manufactured by Keyence Corporation)

Analysis was performed with accompanying analysis software of the devices.

UV-visible Absorption Spectrum: Black Quartz Cell for Spectroscopic Measurement (optical path length: 10 mm) Fluorescence Measurement: a fully transparent synthetic quartz cell (optical path length: 10 mm) and a Starna triangular quartz cell SF3 were used.

A solid measurement unit was used for the solid sample. The sample applied to a cover glass was sandwiched and immobilized with quartz plates to perform measurement.

3D fluorescence spectra were measured at an excitation wavelength of 310 to 800 nm, a data interval of 5 nm, a fluorescence wavelength of 350 to 900 nm, a data interval of 1 nm, and a scan speed of 12000 nm/min, with an ultraviolet cut filter U310 mounted on the excitation side in order to reduce the influence of scattered light and multidimensional light. The bandwidth was 5 nm on the excitation side, and 15 nm on the fluorescence side, with the sensitivity set to low.

The synchro-scanning spectrum is a scan mode in which the excitation spectrometer and the fluorescence spectrometer of a spectrofluorometer are simultaneously allowed to perform scanning with these spectrometers shifted from each other by a predetermined wavelength interval. The measurement was performed by varying the wavelength interval between excitation light and fluorescence within the range of -90 nm to 260 nm. A fluorescence having a wavelength shorter than that of excitation light (i.e., a negative wavelength interval) is detected. At this time, a fluorescence caused by an anti-Stokes shift can be selectively detected. The scanning was set so as to cover the fluorescence wavelength range of 350 to 800 nm, with the data interval set to 0.1 nm, the scanning speed to 600 nm/min, the bandwidth to 15 nm on the excitation side and 20 nm on the fluorescence side, and the sensitivity to low.

The UV-visible absorption spectra were measured with a double monochromator UV-2700. Wavelength scanning (200 to 900 nm) was performed.

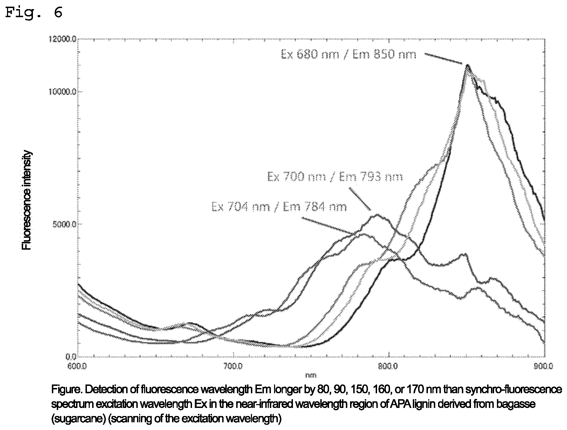

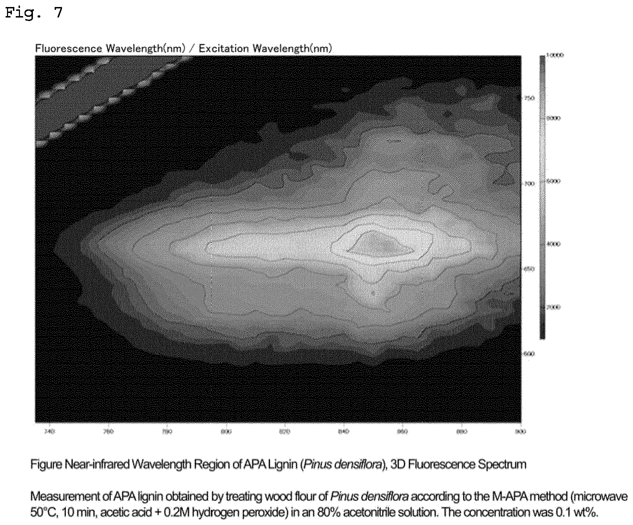

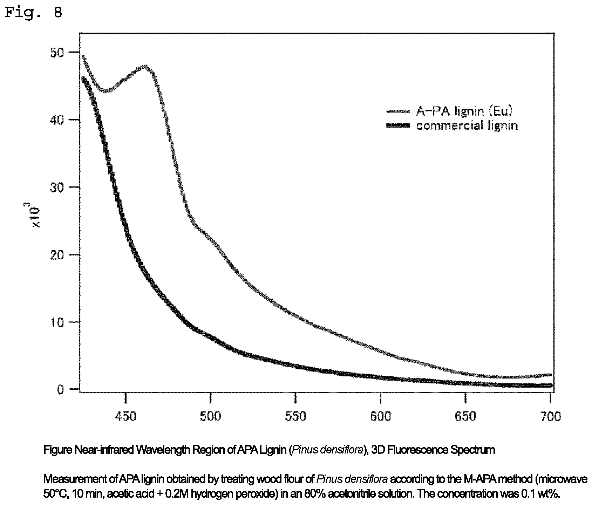

Figs. 6 to 9 show the results. From Fig. 9, the lignin self-assembled body was found to exhibit anti-Stokes fluorescence.

### Reference Test Example 4: Measurement of UV Absorption

The UV absorption properties of the lignin and the lignin-hemicellulose copolymers used in Reference Test Example 2 were measured. Specifically, the samples were individually dissolved in ethanol to 0.005 w/v%, and the UV absorption spectra of the obtained (transparent) solutions were measured with a spectrophotometer (UV-2700 manufactured by Shimadzu Corporation, optical path length: 1 cm, quartz cell).

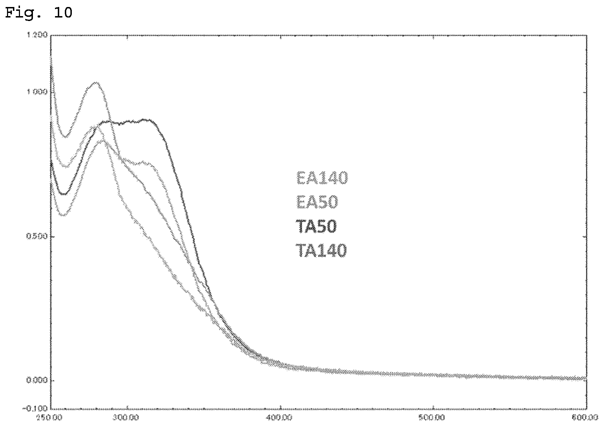

Fig. 10 shows the results. The results indicate that the lignin and lignin-hemicellulose copolymers obtained by the method of the present invention exhibited excellent UV absorption properties.

### Example 1. Preparation of Hollow Spherical Particles 1

The lignin used in Reference Test Example 2 was used as a sample to prepare hollow spherical particles, which are a self-assembled body. Specifically, the preparation was performed as described below.
(1) 20 mg of a lignin sample (dry powder) was dissolved in 1.0 mL of DMSO to prepare a 2% w/v lignin solution.
(2) 0.1 mL of 2% w/v lignin stock solution was added to 0.9 mL of 80% v/v ethanol solution, and the mixture was stirred and mixed uniformly.
(3) 3 mL of water was gradually added dropwise while stirring to dilute the mixture. The dilution rate can be changed. The formation of hollow spherical particles began at an organic solvent concentration of 40% or less, and a stable dispersion state was observed at 5 to 30%.

Alternatively, hollow spherical particles were obtained by a method employing the following step (4) instead of step (3).

(4) 1 mL of an alcohol-water solvent was slowly added dropwise while stirring. The alcohol-water solvent used was a 30% ethanol solution or a 10% n-butanol solution. Thereafter, the solvent was distilled off by gently spraying the solution surface with nitrogen gas, or by drying in air for evaporation, or under reduced pressure. The formation of hollow spherical particles began at an organic solvent concentration of 40% or less, and a stable dispersion state was observed at 5 to 30%.

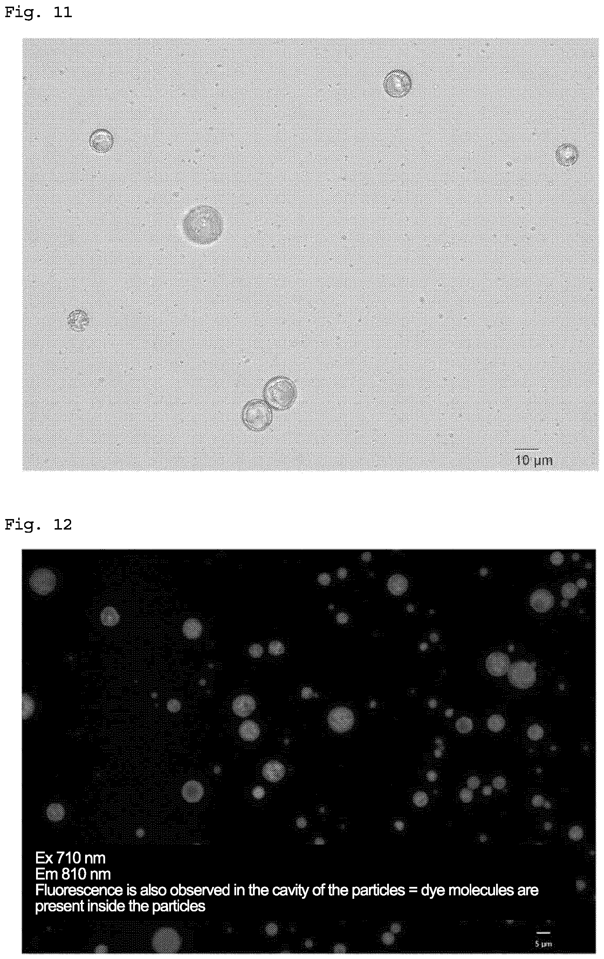

Fig. 11 shows a bright-field image of the resulting hollow spherical particles. Relatively large particles (particle size: 5 µm or more) and relatively small particles (particle size: less than 5 µm) were confirmed.

In addition, the particle size and film thickness of one relatively large particle were measured, and the volume occupied by the cavity of the hollow spherical particle (void ratio) was calculated. Specifically, Keyence All-in-One Fluorescence Microscope BZ-X810 was used to capture fluorescence images using a 100x objective lens in sectioning mode, and the particle size and film thickness were measured from the resulting image data using Keyence analysis software. The void ratio (unit: %) was calculated by the formula: void ratio = (volume of cavity portion of hollow spherical particle (excluding film portion)/total volume of hollow spherical particle) × 100. As a result, the particle size was 21.1 µm, the film thickness was 635 nm, and the void ratio was 83%.

### Example 2. Preparation of Hollow Spherical Particles

### Encapsulating Drug 1

The lignin used in Reference Test Example 2 was used as a sample to prepare hollow spherical particles encapsulating a drug. Specifically, the preparation was performed as described below.

A 0.2% w/v lignin solution (in 80% ethanol) was mixed with 1.0 mL of 0.1% w/v near-infrared fluorescent dye DiR (DiIC18(7)) in 80% ethanol solution while stirring. Water or an alcohol-water solvent was added dropwise with stirring and mixed to reduce the organic solvent concentration in the same manner as in Example 1, thereby forming hollow spherical particles.

Fig. 12 shows an image observed by a fluorescent microscope. Particles exhibiting near-infrared fluorescence derived from the encapsulated dye were observed.

### Example 3. Preparation of Hollow Spherical Particles 2

The lignin used in Reference Test Example 2 was used as a sample to prepare hollow spherical particles. Specifically, the preparation was performed as described below.

A 2.5 wt% solution (30% ethanol) of lignin (derived from *eucalyptus*) was prepared and stirred to obtain a uniform solution, which was used as the stock solution. To 100 µL of the stock solution, 200 µL of a 50% organic solvent (ethanol, 1-propanol, allyl alcohol, 2-propanol, or 1-butanol) was gradually added with stirring to obtain a uniform solution. 1.7 mL of water was gradually added with stirring to obtain a uniform solution. The resulting liquid (final concentration 0.125 wt%, organic solvent 5% (containing 0.015% ethanol)) was subjected to nanoparticle measurement by DLS. For DLS measurement, 2 mL of a sample was placed in a disposable cell for spectroscopy using Multi-Sample Nanoparticle Size Measurement System nanoSAQLA (produced by Otsuka Electronics), and the particle size was measured by the dynamic light scattering method. The measurements were performed three times for each, and the average value was calculated. The measurement temperature was 25°C, and the solvent refractive index was 1.33.

The results are shown in Table 9.

**Table 9**

| Organic solvent | Average particle size [nm] (Cumulant) | Polydispersity index |
|---|---|---|
| Ethanol | 146.5 | 0.122 |
| 1-Propanol | 89.2 | 0.163 |
| Allyl alcohol | 89.1 | 0.138 |
| 2-Propanol | 94.5 | 0.166 |
| 1-Butanol | 214.6 | 0.219 |

In addition, the same tests as above were conducted using lignin derived from other tree species (*Pinus densiflora, Chamaecyparis obtusa, Cryptomeria japonica, Fagus crenata,* and bamboo) (lignin obtained by the same method as in Reference Test Example 2), and similar results were obtained for the average particle size and polydispersity index.

### Reference Test Example 5. Quantification of Hydroxyl Groups

The lignin used in Reference Test Example 2 was used as a sample to quantify hydroxyl groups. In addition, Comparative Examples 1 and 2 from documents were cited to show the quantitative values of hydroxyl groups.

The solvent used in this analysis was a mixture of dehydrated pyridine and CDCl₃ (volume ratio: 1.6/1). A standard derivatization procedure is as follows. 20 mg of a sample was added and dissolved in 400 µL of the solvent. To this, 100 µL (5.3 µmol) of N-hydroxy-1,8-naphthalimide solution (concentration: 11.4 mg/mL) was added as an internal standard, and further 100 µL of tris(2,4-pentanedionato)-chrome(III) [Cr(acac)3] (solution concentration: 5 mg/mL) was added as a relaxation reagent. The mixture was stirred in the dark, and 100 µl of 2-chloro-4,4,5,5-tetramethyl-1,3,2-dioxaphospholane (TMDP) was added, followed by stirring at room temperature for 3 hours to carry out the reaction.

The derivatized samples were subjected to 31P NMR measurement using 400 MHz NMR, produced by JEOL Ltd. Various hydroxyl groups were quantified from the peak integrated values of the obtained 31P NMR spectra. Typically, the peaks at 140.3-144.5 (syringyl-OH, condensed OH), 138.3-140.5 (guaiacyl-OH), and 137.0-138.5 ppm (p-hydrolyphenyl-OH) were assigned to phenolic hydroxyl groups (phenolic-OH), the peaks at 145.0-150.0 ppm to alcoholic hydroxyl groups (aliphatic-OH), and the peaks at 133.6-136.0 ppm to carboxyl hydroxyl groups (carboxylic-OH). The integrated value of alcoholic hydroxyl groups relative to the total integrated value of these peaks, and the integrated value of phenolic hydroxyl groups relative to alcoholic hydroxyl groups were calculated.

The results are shown in Table 10.

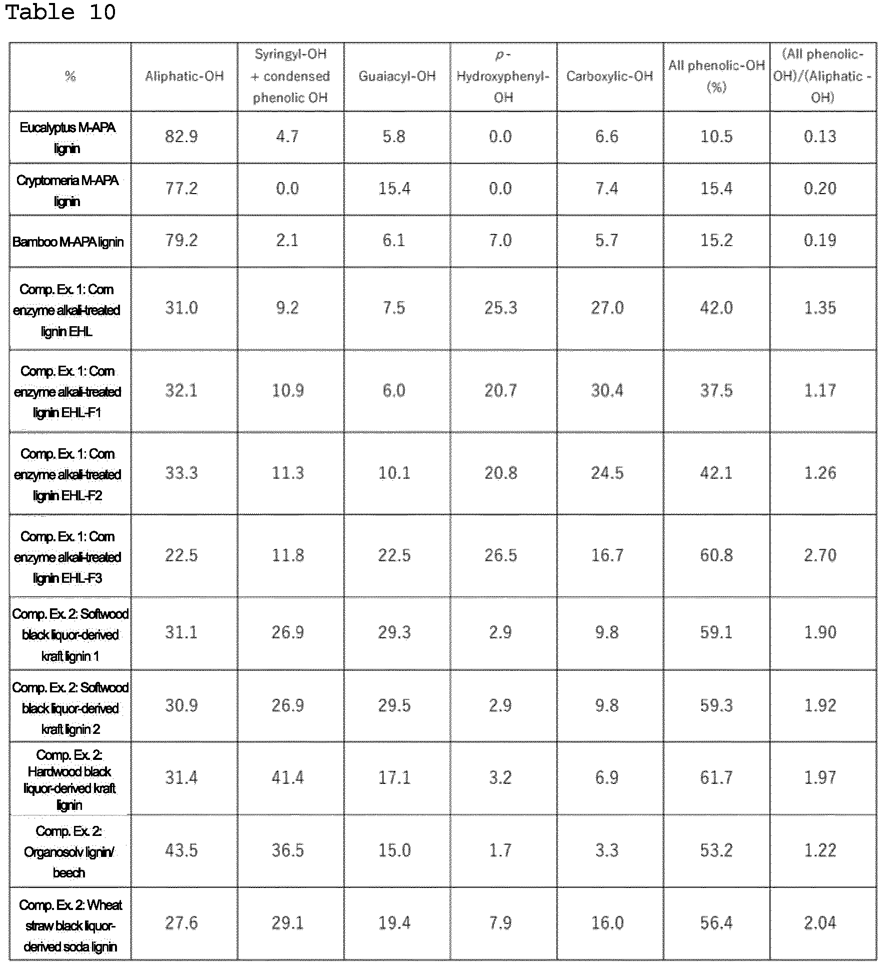

The data of Comparative Example 1 were created from Table 1. Functional Groups contents of EHL and Its Three Fractions Based on the 31P NMR Spectra Results in the reference paper (T. Pang et al., Lignin Fractionation for Reduced Heterogeneity in Self-Assembly Nanosizing: Toward Targeted Preparation of Uniform Lignin Nanoparticles with Small Size, ACS Sustainable Chem. Eng. 2020, 8, 24, 9174-9183 (https://pubs.acs.org/doi/10.1021/acssuschemeng.0c02967).

The data of Comparative Example 2 were cited from the data of Table 2, 162 MHz (HF) ³¹P NMR analysis data in the reference paper (J.F. Araneda et al., Analysis of lignins using 31P benchtop NMR spectroscopy: quantitative assessment of substructures and comparison to high-field NMR, Can. J. Chem. 2022, 100, 799-808 (http://dx.doi.org/10.1139/cjc-2022-0041). However, the peaks at 144.5-140.3 ppm were listed as "Syringyl OH + condensed phenolic OH." In the case of softwood, which does not contain syringyl OH, only condensed phenolic OH is shown.

## Claims

1. A hollow spherical particle that is a self-assembled body of lignin, the lignin having a content of β-O-4 ether type structure of 50% or more and a solubility in 60 to 80% ethanol at 25°C of 2 w/v% or more.

2. The hollow spherical particle according to claim 1, wherein the lignin has a number average molecular weight (Mn) of 0.7 to 9 kDa, or the lignin has a weight average molecular weight (Mw) of 1 to 10 kDa.

3. The hollow spherical particle according to claim 1 or 2, which has a number average particle size of 100 µm or less.

4. The hollow spherical particle according to any one of claims 1 to 3, which has a number average particle size of 50 nm or more.

5. The hollow spherical particle according to any one of claims 1 to 4, which has a void ratio of 60% or more.

6. The hollow spherical particle according to any one of claims 1 to 5, which has a film thickness of 1 nm to 4000 nm.

7. The hollow spherical particle according to any one of claims 1 to 6, comprising an active ingredient.

8. The hollow spherical particle according to claim 7, wherein the active ingredient is at least one member selected from the group consisting of dyes, agricultural chemicals, fertilizers, pharmaceuticals, cosmetic ingredients, fragrances, fluorescent agents, paints, proteins, nucleic acids, lipids, hormones, sugars, glycosides, surfactants, adhesives, vitamins, coenzymes, minerals, cells, organelles, low-molecular-weight compounds, high-molecular-weight compounds, metal complexes, liposomes, and hydrogels.

9. The hollow spherical particle according to any one of claims 1 to 8, wherein a liquid, a gas, or a solid (metal as a type) is enclosed therein.

10. The hollow spherical particle according to claim 9, for use as a heat-insulating material, a compatibilizer, a catalyst, or a sensitizer.

11. The hollow spherical particle according to any one of claims 1 to 10, wherein the lignin has a sulfur atom content of 0.15% or less.

12. The hollow spherical particle according to any one of claims 1 to 11, wherein the lignin has a content of non-natural condensed structure of 1% or less.

13. The hollow spherical particle according to any one of claims 1 to 12, wherein the lignin has a ratio of phenolic hydroxyl groups to alcoholic hydroxyl groups of 0.8 or less.

14. The hollow spherical particle according to any one of claims 1 to 13, which exhibits long-wavelength fluorescence.

15. The hollow spherical particle according to claim 14, wherein the long-wavelength fluorescence is fluorescence at 450 nm or more.

16. An ultraviolet absorber comprising the hollow spherical particle according to any one of claims 1 to 15.

17. A light resistance imparting agent comprising the hollow spherical particle according to any one of claims 1 to 15.

18. An anti-Stokes fluorescent agent comprising the hollow spherical particle according to any one of claims 1 to 15.
